(19) Europäisches Patentamt
European Patent Office
Office européen des brevets

(11) Veröffentlichungsnummer: **0 028 779**
**A1**

# (12) EUROPÄISCHE PATENTANMELDUNG

(21) Anmeldenummer: **80106707.5**

(22) Anmeldetag: **31.10.80**

(51) Int. Cl.[3]: **C 11 D 3/384**
C 11 D 1/02, A 61 K 7/50

(30) Priorität: **08.11.79 DE 2945100**

(43) Veröffentlichungstag der Anmeldung:
**20.05.81 Patentblatt 81/20**

(84) Benannte Vertragsstaaten:
**AT BE CH DE FR GB IT LI NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Fischer, Herbert, Dr.**
**Kamper Weg 139**
**D-4000 Düsseldorf 12(DE)**

(72) Erfinder: **Scheuermann, Fanny geb. Esano-Solomon**
**Volmerswerther Strasse 66**
**D-4000 Düsseldorf(DE)**

(72) Erfinder: **Hase, Christian, Dr.**
**Millrather Weg 29**
**D-4006 Erkrath 1(DE)**

(72) Erfinder: **Krause, Horst-Jürgen, Dr.**
**Am Nettchesfeld 22**
**D-4000 Düsseldorf 13(DE)**

(54) **Neuartige Tenside, deren Herstellung und Verwendung in hautfreundlichen Spül-, Wasch- und Reinigungsmitteln sowie diese enthaltende hautfreundliche, insbesondere kosmetische Zubereitungen.**

(57) Neuartige Tenside auf Basis von Salzen anionischer Tenside, die als Gegenion ein durch Aminolyse von Eiweißkörpern mittels aliphatischer, mehrbasischer Amine gewonnenes, stark basisches Proteinaminolysat enthalten, deren Herstellung und Verwendung sowie diese enthaltende hautfreundliche Wasch-, Spül- und Reinigungsmittel, insbesondere kosmetische Zubereitungen.

EP 0 028 779 A1

HENKEL KGaA
ZR-FE/Patente
z-sü

# P a t e n t a n m e l d u n g

D 5993 EP

"Neuartige Tenside, deren Herstellung und Verwendung in hautfreundlichen Spül-, Wasch- und Reinigungsmitteln sowie diese enthaltende hautfreundliche, insbesondere kosmetische Zubereitungen"

---

Gegenstand der Erfindung sind neuartige Tenside auf Basis von Salzen anionischer Tenside, insbesondere Alkylsulfate und Alkylethersulfate, die als Gegenion ein basisches Proteinaminolysat enthalten, deren Herstellung und Verwendung in hautfreundlichen Spül-, Wasch- und Reinigungsmitteln, sowie diese enthaltende hautfreundliche, insbesondere kosmetische Zubereitungen.

Die Bemühungen keratinhaltiges Material wie Haut und Haare vor den schädlichen Wirkungen von Detergentien zu schützen, wurden bereits vor langer Zeit aufgenommen, ohne daß es bis heute zu einer völlig befriedigenden Lösung gekommen wäre. Es wurden inzwischen zahlreiche Möglichkeiten zur Lösung dieser Probleme vorgeschlagen, wobei für die Aniontenside, die am verbreitesten eingesetzte Tensidklasse, insbesondere zwei unterschiedliche Lösungswege intensiv bearbeitet wurden. Der erste Lösungsweg besteht in dem Zusatz von die Hautfreundlichkeit verbessernden Produkten zu den Aniontenside, wie insbesondere Alkylsulfate oder Alkylethersulfate in Form ihrer Alkali-, Amin- oder Alkylolaminsalze enthaltenden Spül-, Wasch- und Reinigungsmitteln. Als solche, die Hautfreundlichkeit verbessernden Produkte, finden in erster Linie mehr oder weniger chemisch modifizierte Eiweißkörper jedweder

Provenienz Verwendung. Der zweite Lösungsweg zur Verbesserung der Hautfreundlichkeit von Aniontensiden, insbesondere von Alkylsulfaten und Alkylethersulfaten ist die Verwendung anderer Gegenionen anstelle von Natrium. Hierbei haben sich insbesondere die Di- und Triethanolaminsalze der Alkyl- und Alkylethersulfate durchsetzen können, die hautschonendere Eigenschaften aufweisen, als die entsprechenden Natriumsalze.

Es wurde nun gefunden, daß neuartige Tenside auf Basis von Salzen anionischer Tenside, die als Gegenion ein durch Aminolyse von Eiweißkörpern mittels aliphatischer mehrbasischer Amine gewonnenes, stark basisches Proteinaminolysat der allgemeinen Formel

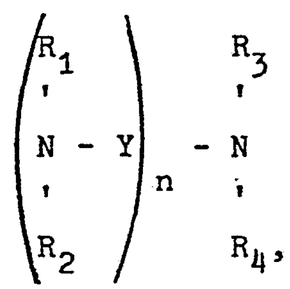

in der

$R_1$ Wasserstoff oder einen Peptidrest bedeutet, mit der Maßgabe, daß wenigstens einer der Reste $R_1$ ein Peptidrest sein muß, der über die Carboxylgruppe des Eiweißrestes verknüpft ist und ein Molgewicht von 200 bis <5 000 besitzt,

Y einen Alkylenrest mit 1 - 4 Kohlenstoffatomen,

$R_2$, $R_3$ und $R_4$, die gleich oder verschieden sein können und Wasserstoff, einen Alkylrest oder Hydroxyalkylrest mit 1 - 4 Kohlenstoffatomen, der gegebenenfalls durch eine Aminogruppe substituiert sein kann,

n eine Zahl von 1 bis 3 /3

darstellen, enthalten, sich durch besondere hautschonende Eigenschaften auszeichnen.

Besondere Bedeutung kommt dabei denjenigen neuartigen Tensiden zu, deren als Gegenion dienendes Proteinaminolysat einen Peptidrest mit einem Molgewicht von 500 bis 1 000 besitzt.

Als saure anionische Tenside, die durch Neutralisation mit den Proteinaminolysaten zu den neuartigen Tensiden führen, sind in erster Linie organische Schwefelsäurereaktionsprodukte zu nennen, die in ihrem Molekül neben einer Sulfonsäure- oder Schwefelsäureestergruppe eine Alkylgruppe mit 8 bis 22 Kohlenstoffatomen, eine Alkylbenzolgruppe mit 9 bis 15 Kohlenstoffatomen in der Alkylgruppe, eine Alkylglycerylethergruppe mit höherem Alkylrest, eine Fettsäuremonoglyceridgruppe, eine Alkylphenolethylenoxidethergruppe mit 1 bis 12 Ethylenoxideinheiten und einem Alkylrest mit 8 bis 18 Kohlenstoffatomen, eine Alkylethylenoxidethergruppe mit 1 bis 30 Ethylenoxideinheiten und einem Alkylrest mit 8 bis 24 Kohlenstoffatomen enthalten. Besondere Bedeutung kommt dabei den Alkylschwefelsäuremonoestern, beziehungsweise Alkyletherschwefelsäuremonoestern mit einer Alkylkette von 8 bis 18 Kohlenstoffatomen zu.

Als Ausgangsproteine, die nach entsprechender Behandlung die erfindungsgemäß einzusetzenden Proteinaminolysate liefern, können alle natürlichen, synthetischen oder biosynthetischen Proteine dienen wie Keratin, Fibrinogen, Hauteiweiß, Gelatine, Molkeneiweiß, Casein, Sojaprotein, Erdnußprotein, Baumwollsamenprotein, Polylysin, Proteine, die aus Mikroorganismen erhalten werden, dienen. Besondere Bedeutung als Ausgangsproteine kommt dabei der Gelatine, dem Casein und dem Hauteiweiß zu.

/4

Die Herstellung der erfindungsgemäßen neuartigen Tenside erfolgt dadurch, daß man das als Ausgangsmaterial dienende Protein mit einer wäßrigen Lösung des im Überschuß eingesetzten aliphatischen, mehrbasischen Amins über längere Zeit bei erhöhter Temperatur von ca. 70 - 100° C intensiv durcharbeitet, das erhaltene Aminolysat ausfällt und von überschüssigem Amin befreit und anschließend das verbleibende hochbasische Proteinaminolysat mit der äquivalenten Menge an Alkylschwefelsäuremonoester beziehungsweise Alkyletherschwefelsäuremonoester neutralisiert. Als zur Aminolyse des Proteins zu verwendende aliphatische,mehrbasische Amine sind Alkylendi-, Tri- und Tetramine zu nennen, wobei den Produkten Ethylendiamin und Diethylentriamin die größte Bedeutung zukommt.

Bevorzugte erfindungsgemäße Tenside sind demnach Neutralsalze von Alkylschwefelsäuremonoestern, beziehungsweise Alkyletherschwefelsäuremonoestern, deren Alkylkette 8 - 18 Kohlenstoffatome besitzt mit Proteinaminolysaten, die durch Aminolyse von Gelatine, Casein oder Hauteiweiß mit Ethylendiamin oder Diethylentriamin erhalten wurden.

Ein weiterer Gegenstand der Erfindung ist die Verwendung der erfindungsgemäßen neuartigen Tenside in hautfreundlichen Spül-, Wasch- und Reinigungsmitteln, insbesondere in kosmetischen Zubereitungen. Als derartige hautfreundliche Reinigungsmittel sind neben Geschirrspülmitteln, Waschmitteln für die kleine Wäsche im Handwaschbecken sowie in erster Linie kosmetische Reinigungsmittel, wie Badezusätze, Duschbadzusätze und Haarshampoos, zu nennen. Die erfindungsgemäßen neuartigen Tenside werden in den genannten Produkten anstelle der bisher eingesetzten Tenside in üblicher Weise verwendet.

Spül-, Wasch- und Reinigungsmittel, insbesondere kosmetische Zubereitungen, die die erfindungsgemäßen Tenside enthalten, stellen einen weiteren Gegenstand der Erfindung dar. Der Gehalt dieser hautfreundlichen Geschirrspülmittel, Handwaschmittel, Badezusätze, Duschbadzusätze, Haarshampoos an den erfindungsgemäßen Tensiden bewegt sich im allgemeinen zwischen 10 bis 70 Gewichtsprozent, bezogen auf die gesamte Zubereitung. Daneben enthalten die Mittel die in derartigen Produkten üblichen Bestandteile. Außer durch ihre herausragende Hautfreundlichkeit erweisen sich die erfindungsgemäßen Tenside als Rohstoffe speziell für die kosmetischen Zubereitungen besonders geeignet, da sie in Wasser relativ niederviskose, hellgelbe Lösungen ergeben, die ausgezeichnet mit den übrigen Bestandteilen verträglich sind, sich sehr gut in kosmetische Formulierungen einarbeiten lassen und lagerstabil sind.

Die nachfolgenden Beispiele sollen den Erfindungsgegenstand näher erläutern, ohne ihn jedoch hierauf zu beschränken.

/6

## Beispiele

### Herstellung der erfindungsgemäßen neuartigen Tenside

150 g des in der nachstehenden Tabelle 1 genannten Eiweißkörpers wurden mit der dort angegebenen Menge Amin und 40 g Wasser in einem Kneter vermischt und 2 Stunden lang bei 95° C geknetet. Anschließend wurde die erhaltene zähe Masse in 1 Liter Isopropanol gegossen. Hierbei fiel das gebildete Aminolysat aus. Dieses wurde durch mehrmaliges Umlösen aus Wasser-Isopropanol gereinigt und anschließend getrocknet. Dabei wurden die in der Tabelle 1 angegebenen Mengen an Aminolysat erhalten.

/7

Tabelle 1

| Produkt | Eiweiß | Ethylendiamin g | Diethylen-triamin g | Ausbeute Aminolysat g | Molgew. Aminolysat | mmol basische Aminogruppen pro g Aminolysat |
|---|---|---|---|---|---|---|
| A | Gelatine | 60 | - | 106 | 650 | 1,68 |
| B | Casein | - | 51 | 119 | 760 | 3,5 |
| C | Gelatine | - | 69 | 119 | 915 | 1,82 |
| D | Casein | - | 69 | 130 | 560 | 3,85 |
| E | Gelatine | 20 | - | 90 | 550 | 1,51 |
| F | Hauteiweiß | 40 | - | 100 | 785 | 1,87 |

Das erhaltene isopropanolfreie Aminolysat wurde 25 %ig in Wasser gelöst und unter einem schnellaufenden Rührwerk mit der in Tabelle 2 angegebenen Menge Alkylethermonoschwefelsäureester neutralisiert. Als Alkylethermonoschwefelsäureester diente der Fettalkohol-$C_{12}$-$C_{14}$-ethermonoschwefelsäureester mit zwei Ethylenoxidgruppen im Molekül. In den erhaltenen erfindungsgemäßen Tensiden sind alle basischen Aminogruppen neutralisiert, die Produkte weisen einen pH-Wert von 6,5 - 7 auf. In der Tabelle 2 ist ferner die Konzentration der erhaltenen wäßrigen Tensidlösung angegeben.

Tabelle 2

| Produkt | g Aminolysat | g Alkylethermonoschwefelsäureester | Konzentration Tensid % |
|---|---|---|---|
| A | 106 | 50 | 34 |
| B | 119 | 120 | 44 |
| C | 119 | 57 | 37 |
| D | 130 | 139 | 41 |
| E | 90 | 36 | 32 |
| F | 100 | 53 | 34 |

/9

Zur Bestimmung der Hautfreundlichkeit der erfindungsgemäßen neuartigen Tenside wurde der Zein-Test durchgeführt. Als Testprodukt diente das Neutralisationsprodukt von Fettalkohol-$C_{12}$-$C_{14}$-etherschwefelsäuremonoester mit dem Aminolysat von Casein + Diethylentriamin vom Molgewicht 670. Als Vergleichssubstanzen dienten Nonylbenzolsulfonat (Na-Salz), Natriumlaurylsulfat und Natriumlaurylethersulfat mit 2 Ethylenoxidgruppen im Molekül.

Die bei dem Zeintest erhaltene Zeinzahl gibt an, wieviel mg Stickstoff in 100 ml einer Tensidlösung enthalten sind, wenn diese 1 Stunde lang bei 35° C mit 2 g Zein versetzt wird. Je niedriger die Zeinzahl, um so besser ist die Hautverträglichkeit des Produktes. Der Zein-Test wird in der nachfolgend beschriebenen Weise durchgeführt:

1. <u>Ansetzen der Stammlösungen:</u>

1.1. Natronlauge 1/20 n

Die 1/20 n Natronlauge wird mit Benzoesäure (DAB 6) eingestellt. Dazu werden circa 40 bis 50 mg Benzoesäure in einen 100 ml Erlenmeyerkolben eingewogen und in ungefähr 25 ml neutralisiertem Ethylalkohol gelöst. Zum Neutralisieren wird der Alkohol mit einigen Tropfen verdünnter Natronlauge gegen Phenolphthalein bis zur bleibenden Rosafärbung versetzt. Die gelöste Benzoesäure wird nun mit der 1/20 n Natronlauge bis zur bleibenden Rosafärbung titriert und der Faktor berechnet:

1 ml 1/20 n NaOH = 6,1 mg Benzoesäure.

1.2. Schwefelsäure 1/20 n

Aus einer Mikrobürette läßt man 10 ml der 1/20 n Schwefelsäure in einen 100 ml Erlenmeyerkolben einlaufen, gibt 5 Tropfen Tashiro-Indikator hinzu und bringt die Vorlage am Destillierapparat an. In den Destillierkolben gibt man 20 ml 25 %ige Natronlauge, verdünnt mit destilliertem Wasser bis zur Hälfte des Volumens und destilliert 7 bis 10 Minuten. Die Vorlage wird nun abgenommen, und, nachdem mit etwas destilliertem Wasser das in Schwefelsäure eintauchende Glasrohr abgespült wurde, titriert man mit 1 /20 n Natronlauge von violett auf blaugrün. Der erhaltene Wert ist der Blindwert, der auch gleichzeitig eine Kontrolle des Destillier-Apparates ist und regelmäßig bestimmt wird. Diese Zahl wird der Berechnung des Stickstoffgehaltes zugrunde gelegt.

1.3. Tashiro-Indikator

0,24 g Methylrot in 300 ml Ethanol und 0,40 g Methylenblau in 400 ml 50 %igem Ethanol.

1.4. 25 %ige Natronlauge aus NaOH-Plätzchen.

1.5. Das zu prüfende Tensid wird als x-prozentige Lösung mit destilliertem Wasser angesetzt und auf pH 7 eingestellt.

2. Aufbereitung des Zein:

Das verwendete Zein aus Mais (MG 20.000 - 30.000; ~98,5 % Protein) wird im Vakuumtrockenschrank bei maximal 40° C bis zur Gewichtskonstanz getrocknet.

3. Durchführung des Tests:

In eine 50 ml-Enghalsflasche mit Schraubverschluß werden 40 ml Tensidlösung pipettiert und in einem Wasserbad auf 35° C temperiert. 2 g Zein werden auf einer Spielkarte genau eingewogen und durch einen Trichter aus Fotopapier schnell in die Flasche geschüttet und sofort 25 mal per Hand kräftig hin- und hergeschüttelt, damit sich das Zein gut verteilt und nicht zusammenballt.

In einem Wackerapparat, dessen Lufttemperatur auf 35° C eingestellt ist und der für diesen Zweck geeignete Stahleinsätze mit Federn enthält, können mehrere Flaschen eingespannt werden, die nun 1 Stunde bei 5 U/min. rotieren. Anschließend wird ein aliquoter Teil der Lösung (ungefähr 25 ml) in einem Zentrifugenbecher aus V2A-Stahl eingewogen und 30 Minuten bei 2600 U/min. zentrifugiert. Nun werden ungefähr 10 ml durch einen Faltenfilter (Schleicher & Schüll, Nr. 597 1/2 Schwarzband Ø 5,5 cm) in einen 50 ml-Erlenmeyerkolben filtriert. Der Stickstoffgehalt dieser Lösung wird durch eine Doppelbestimmung nach der Mikro-Kjeldahl-Methode ermittelt.

1 ml der gelben Lösung (bei sehr schwach gefärbten Lösungen werden 2 bis 5 ml verwendet) wird in einem 100 ml Kjeldahl-Kolben mit einer kleinen Menge Selen-Reaktionsgemisch und ungefähr 3 ml konzentrierter Schwefelsäure versetzt. Der Kolben wird in das Elektro-Thermal-Gerät gebracht (die Kolben müssen außen sauber und trocken sein, ebenso darf keine Lösung in die Heizaggregate tropfen) und in der Hitze ungefähr 2 1/2 - 3 Stunden aufgeschlossen, bis die Flüssigkeit wäßrig klar ist.

/12

Die abgekühlte Lösung wird nun in einen Destillierapparat überführt, mit 20 ml 25 %iger Natronlauge und einigen Tropfen Phenolphthalein versetzt, wobei darauf geachtet werden muß, daß die Probelösung auch wirklich alkalisch ist. Der Kolben darf nur ungefähr bis zur Hälfte gefüllt sein. Nun wird mit Wasserdampf in 7 - 10 Minuten (eventuell länger, mit pH-Papier prüfen) das Ammoniak in eine Vorlage mit 10 ml 1/20 n Schwefelsäure und circa 10 ml destilliertem Wasser, überdestilliert. Durch Rücktitration der unverbrauchten Schwefelsäure in der Vorlage mit 1/20 n Natronlauge gegen Tashiro-Indikator wird der Säureverbrauch durch das überdestillierte Ammoniak und daraus die Stickstoffaufnahme berechnet:

$$1\ \text{ml}\ 1/20\ \text{n}\ H_2SO_4 \triangleq 0{,}7\ \text{mg N}.$$

Bei stickstoffhaltigen Tensiden wird der Gehalt an Stickstoff ebenfalls nach der Mikro-Kjeldahl-Methode bestimmt und bei der Ausrechnung vom Endergebnis abgezogen.

Bei dem Zein-Test wurden die in nachstehender Tabelle 1 aufgeführten Zein-Zahlen ermittelt, denen die sehr gute Hautfreundlichkeit der erfindungsgemäßen Tenside zu entnehmen ist.

/13

Tabelle 1

| S u b s t a n z | Zein - Zahl |
|---|---|
| Nonylbenzolsulfonat 20 %ig | 620 |
| Natriumlaurylsulfat 20 %ig | 535 |
| Natriumlaurylethersulfat 20 %ig | 288 |
| Natriumlaurylethersulfat 5 %ig | 113 |
| Natriumlaurylethersulfat 1 %ig | 13 |
| Erfindungsgemäßes Neutralisationsprodukt 20 %ig | 25 |

Nachfolgend werden noch einige Beispiele für Reinigungsmittel mit einem Gehalt an den erfindungsgemäßen Tensiden aufgeführt:

Reinigungsmittel

20 % Produkt A (Neutralisationsprodukt Fettalkohol-$C_{12}$-$C_{14}$-ethermonoschwefelsäureester mit Aminolysat Gelatine/Ethylendiamin)
25 % Trikaliumorthophosphat
0,1 % Parfümöl
Rest Wasser.

/14

Reinigungsmittel

15 % Produkt B (Neutralisationsprodukt Fettalkohol-$C_{12}$-$C_{14}$-ethermonoschwefelsäureester mit Aminolysat Casein/Diethylentriamin)
10 % Harnstoff
5 % Ethanol
0,1 % Parfümöl
Rest Wasser.

Schaumbad

35 % Produkt C (Neutralisationsprodukt Fettalkohol-$C_{12}$-$C_{14}$-ethermonoschwefelsäureester mit Aminolysat Gelatine/Diethylentriamin)
5 % Kokosfettsäurediethanolamid
3 % Parfümöl
57 % Wasser.

Duschbad

30 % Produkt D (Neutralisationsprodukt Fettalkohol-$C_{12}$-$C_{14}$-ethermonoschwefelsäureester mit Aminolysat Casein/Diethylentriamin)
3 % Kokosfettsäuremonoethanolamid
5 % Kokosfettsäurediethanolamid
2 % Parfümöl
60 % Wasser.

/15

Baby-Schaumbad

60 % Produkt E (Neutralisationsprodukt Fettalkohol-$C_{12}$-$C_{14}$-ethermonoschwefelsäureester mit Aminolysat Gelatine/Ethylendiamin)
15 % Capryl-/Caprinsäuretriglycerid
3 % Kamillenextrakt
1 % Parfümöl
21 % Wasser.

Haar-Shampoo

20 % Produkt F (Neutralisationsprodukt Fettalkohol-$C_{12}$-$C_{14}$-ethermonoschwefelsäureester mit Aminolysat Hauteiweiß/Ethylendiamin)
6 % Kokosfettsäuremonoethanolamid
2 % Parfümöl
72 % Wasser.

/16

"Neuartige Tenside, deren Herstellung und Verwendung in hautfreundlichen Spül-, Wasch- und Reinigungsmitteln sowie diese enthaltende hautfreundliche, insbesondere kosmetische Zubereitungen"

Patentansprüche:

1. Neuartige Tenside auf Basis von Salzen anionischer Tenside, dadurch gekennzeichnet, daß sie als Gegenion ein durch Aminolyse von Eiweißkörpern mittels aliphatischer, mehrbasischer Amine gewonnenes, stark basisches Proteinaminolysat der allgemeinen Formel

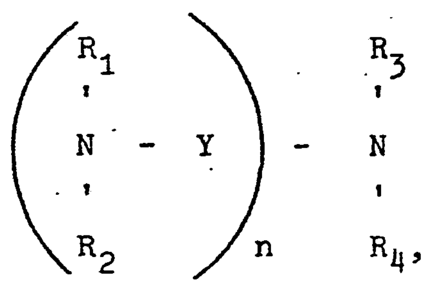

in der

$R_1$ Wasserstoff oder einen Peptidrest bedeutet, mit der Maßgabe, daß wenigstens einer der Reste $R_1$ ein Peptidrest sein muß, der über die Carboxylgruppe des Eiweißrestes verknüpft ist und ein Molgewicht von 200 bis <5000 besitzt,

Y einen Alkylenrest mit 1 - 4 Kohlenstoffatomen,

$R_2$, $R_3$ und $R_4$, die gleich oder verschieden sein können und Wasserstoff, einen Alkylrest oder Hydroxyalkylrest mit 1 - 4 Kohlenstoffatomen, der gegebenenfalls durch eine Aminogruppe substituiert sein kann,

n eine Zahl von 1 bis 3

darstellen, enthalten.

/17

2. Neuartige Tenside nach Anspruch 1, dadurch gekennzeichnet, daß der Peptidrest $R_1$ ein Molgewicht von 500 bis 1000 besitzt.

3. Neuartige Tenside nach Anspruch 1 und 2, dadurch gekennzeichnet, daß als anionische Tenside Alkylschwefelsäuremonoester, beziehungsweise Alkyletherschwefelsäuremonoester mit einer Alkylkette von 8 bis 18 Kohlenstoffatomen dienen.

4. Neuartige Tenside nach Anspruch 1 - 3, dadurch gekennzeichnet, daß die als Gegenion dienenden Proteinaminolysate von Gelatine, Hauteiweiß oder Casein abgeleitet sind.

5. Neuartige Tenside nach Anspruch 1 - 4, dadurch gekennzeichnet, daß die als Gegenion dienenden Proteinaminolysate durch Aminolyse mittels Ethylendiamin und Diethylentriamin erhalten werden.

6. Herstellung der neuartigen Tenside gemäß Anspruch 1 - 5, dadurch gekennzeichnet, daß man das als Ausgangsmaterial dienende Protein mit einer wäßrigen Lösung des im Überschuß eingesetzten aliphatischen,mehrbasischen Amins über längere Zeit bei einer Temperatur von circa 70 - 100° C intensiv durcharbeitet, das Aminolysat ausfällt und von überschüssigem Amin befreit und das erhaltene hochbasische Proteinaminolysat mit der äquivalenten Menge des sauren anionischen Tensids neutralisiert.

7. Herstellung der neuartigen Tenside nach Anspruch 6, dadurch gekennzeichnet, daß zur Neutralisation als anionisches Tensid Alkylschwefelsäuremonoester, beziehungsweise Alkyletherschwefelsäuremonoester dienen.

8. Verwendung der neuartigen Tenside nach Anspruch 1 - 5 als alleinigen tensidischen Anteil oder im Gemisch mit anderen Tensiden in hautfreundlichen Wasch-, Spül- und Reinigungsmitteln, insbesondere kosmetischen Zubereitungen.

9. Hautfreundliche Wasch-, Spül- und Reinigungsmittel, insbesondere kosmetische Zubereitungen mit einem Gehalt an den neuartigen Tensiden gemäß Anspruch 1 - 5 als alleinigen tensidischen Anteil, beziehungsweise im Gemisch mit anderen Tensiden.

10. Hautfreundliche Wasch-, Spül- und Reinigungsmittel, insbesondere kosmetische Zubereitungen nach Anspruch 9 mit einem Gehalt an den neuartigen Tensiden in einer Menge von 10 - 70 Gewichtsprozent, bezogen auf das gesamte Mittel.

0028779

Europäisches Patentamt

# EUROPÄISCHER RECHERCHENBERICHT

Nummer der Anmeldung

EP 80 10 6707.5

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| P | US - A - 4 195 077 (PROCTER & GAMBLE)<br>* Ansprüche 1, 3, 4; Spalte 8, Zeilen 6 bis 10, Zeilen 36 bis 46; Spalte 9, Zeilen 7, 8 *<br>-- | 1,3,<br>4,8 | C 11 D 3/384<br>C 11 D 1/02<br>A 61 K 7/50 |
| A | DE - A1 - 2 753 850 (COLGATE-PALMOLIVE)<br>* ganzes Dokument *<br>-- | | |
| A | US - A - 4 155 882 (LEVER BROTHERS)<br>* ganzes Dokument *<br>---- | | |

RECHERCHIERTE SACHGEBIETE (Int. Cl.³)

A 61 K 7/00
C 11 D 1/00
C 11 D 3/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 21-01-1981 | SCHULTZE |

EPA form 1503.1 06.78